(19) Europäisches Patentamt
European Patent Office
Office européen des brevets

(11) EP 4 729 044 A1

(12) EUROPEAN PATENT APPLICATION

(43) Date of publication:
22.04.2026 Bulletin 2026/17

(21) Application number: 25207653.4

(22) Date of filing: 09.10.2025

(51) International Patent Classification (IPC):
A61K 8/34 (2006.01)        A61K 8/365 (2006.01)
A61K 8/44 (2006.01)        A61K 8/9789 (2017.01)
A61P 17/10 (2006.01)       A61Q 19/00 (2006.01)

(52) Cooperative Patent Classification (CPC):
A61K 8/345; A61K 8/365; A61K 8/44;
A61K 8/9789; A61P 17/10; A61Q 19/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 09.10.2024 IT 202400022392

(71) Applicant: Keminova Italiana S.B. S.r.l.
25060 Cellatica (BS) (IT)

(72) Inventors:
• VILLA, Carla
  16125 Via Ogerio Pane, 3 - GENOVA (IT)
• CAVIGLIA, Deobra
  17019 Via Alpicella, 13 - VARAZZE (SAVONA) (IT)
• RUSSO, Eleonora
  16148 Via dei Narcisi, 52 - GENOVA (IT)

(74) Representative: Biggi, Cristina
Bugnion S.p.A.
Viale Lancetti 17
20158 Milano (IT)

(54) COSMETIC/PHARMACEUTICAL FORMULATION CONTAINING LAURUS NOBILIS EXTRACT PREPARED WITH NATURAL DEEP EUTECTIC SOLVENTS

(57) The present invention relates to a cosmetic or pharmaceutical formulation based on laurel plant extract (*Laurus nobilis L.*) and its use for the treatment of acne. The invention also relates to the use of an extract of the laurel plant for the preparation of cosmetic formulations, in which the extract is an extract prepared with NADES (*natural deep eutectic solvents*).

The ternary eutectic mixture used to obtain the NADES solvent comprises glycerine or glycerol, lactic acid and at least one of betaine and choline chloride.

The extract of the laurel plant has a phenol content expressed in mg of gallic acid per gram of laurel, between 10 and 100 mg/L, preferably between 20 and 80 mg/L, and further comprises at least one of: octadecanale, preferably in an amount between 8% and 11% by weight; eugenol, preferably in quantities of 5% to 8%; linalool, preferably in quantities of 2 to 8%; terpinen-4-ol, preferably in quantities of 1 to 5%; 1,8-cineole, preferably in quantities of 0.5 to 5%.

EP 4 729 044 A1

**Description**

**FIELD OF THE INVENTION**

The present invention relates to a cosmetic or pharmaceutical formulation based on extract of the laurel plant (*Laurus nobilis L.*) and its use for the treatment of acne. The invention also concerns the use of an extract of the laurel plant for the preparation of cosmetic formulations, in which the extract is an extract prepared with NADES (*natural deep eutectic solvents).*

**STATE OF THE ART**

Laurel (*Laurus nobilis L.*) is an ancient evergreen plant belonging to *the Lauraceae* family, native to the Mediterranean region where it is widely cultivated as an ornamental plant, as a high-value spice crop and for flavouring and fragrance applications. Laurel leaves contain several biologically active compounds (BAS), including norisoprenoids, alkaloids, tocopherols and a large number of polyphenols, including flavonoids, phenolic acids and lignans [1].

Aqueous extracts (from the fruit and leaves) can be used in herbal medicine to treat various neurological and urological disorders [2] and as astringents in dermatology.

The essential oil is commonly used in popular natural remedies (particularly for rheumatism and dermatitis) and in cosmetics for various health properties [3] thanks to its antioxidant, antimicrobial and antiinflammatory effects and its fragrance. For example, it has been successfully incorporated into anti-dandruff products and formulations for the treatment of psoriasis.

Thanks to its biological activity, essential oil from laurel leaves could be considered a natural antioxidant supplement in cosmetics and medicine [4].

The biological properties of *Laurus* are attributed to a plethora of highly bioactive volatile compounds (in essential oil) and phenolic compounds (from other extracts), traditionally recovered by solid-liquid extraction with organic or hydroalcoholic solvents.

The use of eutectic solvents, in particular natural eutectic solvents (NADES), to prepare extracts from biological materials is well known. The application of these extracts in cosmetic formulations is also well known [5].

However, the application of this technique for the extraction of the laurel plant is not known, nor is the use of particular extraction mixtures that allow extracts effective in the treatment of acne to be obtained.

**SUMMARY OF THE INVENTION**

The present invention relates to an extract of the laurel plant (*Laurus nobilis* L.), preferably from laurel leaves, obtained using a NADES solvent as an extraction solvent, which is obtained from or comprises a ternary mixture of molecules.

In particular, the invention relates to a composition comprising a ternary mixture of molecules constituting the eutectic solvent and a mixture of components extracted from the laurel plant, in particular from laurel leaves, said extracted mixture having a phenol content, expressed in mg of gallic acid per gram of laurel, of between 10 and 100 mg/L, preferably between 20 and 80 mg/L.

The invention also relates to a cosmetic or pharmaceutical formulation comprising a composition based on eutectic solvents and laurel extract which has improved antioxidant and antibacterial activity, as well as excellent results in the treatment of acne.

**DETAILED DESCRIPTION OF THE INVENTION**

The NADES eutectic solvent used to prepare the composition of the invention comprises a mixture of hydrogen donors and hydrogen acceptors.

NADES are eutectic solvents that can be easily obtained from naturally occurring molecules, hydrogen bond acceptors (HBAs) paired with hydrogen bond donors (HBDs) that form supramolecular structures with very low melting points.

The NADES eutectic solvent used in the present invention is obtained from a ternary mixture comprising glycerine or glycerol, lactic acid and at least one of betaine and choline chloride. The hydrogen donors are choline chloride and betaine, while the acceptors are lactic acid and glycerol.

In one embodiment, the eutectic mixture comprises: glycerine, lactic acid and betaine.

In another embodiment, the eutectic mixture comprises: glycerine, lactic acid and choline chloride.

Both mixtures have provided good results in terms of activity, as described in more detail below; however, the mixture with betaine instead of choline chloride has a better smell, as the presence of choline chloride gives the composition an amine or 'fishy' smell that may be unpleasant.

The ternary mixture comprises at least one of choline chloride and betaine, glycerol and lactic acid in a weight ratio of between 1:1:1 and 3:1:1, preferably 2:1:1.

The NADES eutectic mixture is used to prepare an extract of the laurel plant, preferably an extract of laurel leaves (*Laurus nobilis L.*).

The extraction is carried out by bringing an appropriate amount of laurel into contact with the ternary eutectic mixture and heating the resulting suspension for an appropriate time.

Preferably, laurel leaves are used that are chopped/crushed before extraction.

The extraction is advantageously carried out at a temperature comprised between 50°C and 100°C, preferably between 60°C and 80°C.

In one embodiment, the laurel leaf suspension in the solvent mixture is heated by microwave.

The heating time is comprised between 5 and 20 minutes using the microwave. If other heating methods are used, the extraction times can be much longer and sometimes lead to the degradation of the laurel leaf matrix and consequently its BAS due to prolonged exposure to heat.

Once heating is complete, the suspension is centrifuged to separate the solid residue from the supernatant.

The supernatant thus obtained is a composition comprising the ternary eutectic mixture and the substances extracted from laurel, in particular from laurel leaves.

This composition has a total phenol content, expressed as mg equivalents of gallic acid per g of fresh laurel leaves (mg GAE/g), comprised between 10 and 100 mg/L, preferably between 20 and 80 mg/L.

In addition to phenols, the laurel extract comprises one or more of the following: octadecanal, preferably between 8% and 11% by weight; eugenol, preferably between 5% and 8%; linalool, preferably between 2% and 8%; terpinen-4-ol, preferably between 1% and 5%; 1,8-cineole, preferably between 0.5 and 5%.

The phenolic content is measured using the Folin-Ciocalteu UV/VIS spectrophotometric method, using gallic acid as standard reference.

The composition thus obtained, based on laurel extract and a ternary eutectic mixture, has been shown to possess antioxidant and antimicrobial activity. In particular, the antimicrobial activity has been demonstrated on both Gram+ and Gram- strains, while in comparison, the extracts obtained using binary mixtures of eutectic solvents, e.g. choline chloride/glycerol and betaine/glycerol, show only partial antimicrobial activity, i.e. only on Gram+ bacterial strains.

In other words, extraction with the ternary mixture yields a mixture of compounds with improved antibacterial activity compared to that achieved by extracting the same biological matrix, i.e. laurel leaves, with a binary mixture.

The composition based on laurel extract and comprising the ternary eutectic mixture not only has antioxidant and antibacterial properties, but also anti-acne properties.

Furthermore, thanks to the presence of eutectic solvents, the composition remains unaltered for long periods of time.

The composition is therefore a ready-to-use active ingredient that can be used without further processing to prepare cosmetic or pharmaceutical formulations.

The composition based on laurel extracts and eutectic mixture is used as it is as an active ingredient for cosmetic or pharmaceutical formulations, such as creams, emulsions, oils, ointments and spray solutions.

In addition to the composition based on laurel extract and eutectic mixture as the active ingredient, the cosmetic or pharmaceutical formulation also comprises excipients commonly accepted in cosmetic practice, such as xanthan gum, sodium benzoate, cetearyl alcohol, and pentylene glycol. The cosmetic or pharmaceutical formulation has antioxidant and antibacterial properties and has been shown to be effective in the treatment of skin acne.

The subject of the invention is therefore the use of the cosmetic or pharmaceutical formulation for the treatment of acne and a method of treating acne that includes a step of topically applying the cosmetic or pharmaceutical formulation to the acne-affected area of a patient in need.

**EXAMPLES**

**Materials and methods**

Microwave apparatus

Multimodal MW cavity prototype, equipped with a specially designed Pyrex reactor, a magnetron operating at 2.45 GHz, two fibre optic probes for temperature measurement and a control unit that allows the management of various process parameters such as emitted power, temperature setpoint and mechanical agitation.

The Thermoscientific UV/VIS spectrophotometer (Evolution 300, Fischer Scientific, GmbH, Schwerte, Germany) was used to record UV spectra and for RSA% and TPC analyses.

GC/MS analyses were performed with a Model 6890N GC coupled to an Agilent 5973 Network benchtop MS (Agilent, Santa Clara, CA, USA) using an Elite-5MS capillary column (5% phenylmethylpolysiloxane) (30 m $\times$ 0.32 mm i.d.) and 0.32 $\mu$m thick film (Agilent, Santa Clara, CA, USA). Identification using the NIST/EPA/NIH mass spectrum database. Version 2.1 Perkin-Elmer Instrument LLC.

The VISIA CR system (Canfield Scientific Inc, Fairfield, NJ, USA) and the PRIMOS CR system (Canfield Scientific, Parsippany, New Jersey) were used for facial image acquisition and data analysis, respectively.

## Reagents

All chemical reagents were supplied by Sigma-Aldrich, Milan.

NADES solvents were prepared fresh, in accordance with the literature [6]. The composition and abbreviations are as follows:

CG = Choline chloride/glycerol 1:2
BG = Betaine/Glycerol 1:2
CGA = Choline chloride/glycerol/lactic acid 2:1:1
BGA = Betaine/Glycerol/Lactic acid 2:1:1

## Plant material

For all experiments, laurel leaves (*Laurus nobilis L.*) were obtained from a local Italian supplier.

Distilled water was used to clean the fresh botanical material in order to remove physical impurities; the samples were then chopped up and stored at -20°C until use.

## Bacterial strains

Six multidrug-resistant (MDR) clinical bacterial strains (four Gram-positive and two Gram-negative) were used in this study (see Table 2): two methicillin-resistant *Staphylococcus aureus* (MRSA), two methicillin-resistant *Staphylococcus epidermidis* (MRSE), one carbapenem-resistant and ceftazidime-avibactam-resistant *Escherichia coli* (CAZ-AVI), one ceftazidime-avibactam-resistant *Escherichia coli* (CAZ), and one carbapenem-resistant *Pseudomonas aeruginosa* through the production of *Klebsiella pneumoniae carbapenemase* (KPC). All strains belonged to a collection obtained from the School of Medical and Pharmaceutical Sciences (University of Genoa) and were identified using VITEK® 2 mass spectrometry (Biomerieux, Florence, Italy) or laser-assisted desorption/ionisation time-of-flight (MALDI-TOF) (Biomerieux, Florence, Italy).

## Procedures

### NADES extraction

1 g of fresh chopped leaves was added to 10 g of previously prepared NADES solvent (CG, CGA, BG, BGA). After 5 minutes of sonication, the mixture was extracted for 5 minutes under microwave activation at a temperature of 75°C. After centrifugation, the supernatant solution was stored at 4°C for single use.

### Total phenolic content (TPC)

The total phenolic content of the samples was evaluated using the Folin-Ciocalteu UV/VIS spectrophotometric method, using gallic acid as a reference standard [7-8]. The TPC was calculated from a calibration curve prepared with standard solutions of gallic acid in concentrations ranging from 20 to 80 mg/L ($R^{(2)}$=0.9988) and the results were expressed as mg equivalents of gallic acid per g of fresh laurel leaves (mg GAE/g). All analyses were performed in triplicate (n = 3) and the values are indicated with their standard deviation (SD).

### Antiradical antioxidant activity (RSA%)

The radical activity of each extract was measured using the DPPH assay, based on the bleaching rate of the stable radical 2,2-diphenyl-1-picrylhydrazyl (DPPH) using Trolox as a reference standard [9], obtaining a linear calibration curve ranging from 20 to 200 mg/L ($R^2$ = 0.9952).

0.1 mL of sample was mixed with 3.9 mL of DPPH methanol solution (65 $\mu$M). The absorbance was measured at 516 nm after being stored for 30 minutes in the dark. The results were calculated as Trolox equivalents in mg/L, and the percentage of antioxidant activity (AA%) was calculated from the ratio of the decreasing absorbance of the sample solution (A0-As) to the absorbance of the blank DPPH solution (A0), as expressed in Eq. 1 [10]. Each analysis was performed in triplicate (n = 3) and the values are indicated with their standard deviations (SD).

$$AA\% = (A0\text{-}As)/A0 \times 100 \qquad\qquad (1)$$

Antimicrobial potential (MIC)

MIC values were determined following the microdilution procedures described by the European Committee on Antimicrobial Susceptibility Testing (EUCAST) [11].

Briefly, after overnight incubation, bacterial cultures were diluted to obtain a standardised inoculum of $1.5 \times 10^8$ CFU/mL. Appropriate aliquots of each suspension were added to 96-well microplates containing the same volumes of 2-fold serial dilutions (from 25% to 0.09% of the test samples to obtain a final bacterial concentration of approximately $5 \times 10^5$ cells/mL). After 24 hours of incubation at 37°C, the lowest percentage amount of sample (v/v) that prevented visible growth was recorded as MIC%. The four NADES not containing leaf extract were used as controls to evaluate the antibacterial activity of the solvents used. All MICs were obtained at least in triplicate and the results were expressed by reporting the modal value, i.e. the one observed most frequently. In case of ambiguous or unclear results, more than three MIC determinations were performed.

GC/MS analysis

The BGA sample (1 g) was diluted with 1.2 ml of ultrapure water and then extracted with n-hexane (3 x 1 ml). The organic phase was dried over anhydrous $Na_2SO_4$ and completely evaporated with a gentle stream of $N_2$. The residue was dissolved in 50 $\mu$l of n-hexane prior to analysis.

Chromatographic separation was performed using a 30 m x 0.32 mm diameter phenylmethylpolysiloxane capillary column with a 0.32 $\mu$m thick film. The carrier gas was helium at a flow rate of 1 ml/min. A $\mu$L aliquot of sample was injected manually in splitless mode. The oven temperature programme consisted of an initial isotherm of 40°C for 5 minutes, followed by a temperature ramp to 260°C at 40 C/min and a final isotherm at this temperature for 10 minutes. The injector and detector temperatures were set at 250°C and 280°C, respectively. Mass spectra were acquired in the range 40-400 amu at 1 scan/sec with an ionising electron energy of 70 eV. The identification of volatile compounds was performed using retention indices (RI) and mass spectra, according to Adams [12], by comparison with a mass spectrum library from the NIST database (NIST/EPA/NIH Mass Spectral Database. Version 2.1 Perkin-Elmer Instrument LLC). The relative amount of each component was expressed as the peak area as a percentage of the total peak areas of the GC/MS analyses of the entire extract.

**In vivo tests**

Subjects and study protocol

Twenty healthy male and female subjects (aged between 18 and 35 years) were recruited for this study who met the following criteria: oily or combination skin with visible acne, including inflammatory and non-inflammatory acne lesions on the skin of the face [13, 14]. During the experimental period, all participants were instructed to apply the Active Cream to one side of the face and the Placebo Cream to the other side, twice a day (morning and evening) for 28 consecutive days. Assessments were carried out at the beginning, after a 3-day treatment period, and after 28 days.

The only differences between the two creams were the presence of NADES extract in the Active Cream and the three individual components of the NADES mixture added separately to the Placebo Cream. The INCI was: Aqua, Caprylic/Capric Triglyceride, Glycerin, Steareth-2, Pentylene Glycol, Betaine, Cetearyl Alcohol, Lactic Acid, Steareth-21, Laurus Nobilis Leaf Extract, Xanthan Gum, Sodium Benzoate, Chlorphenesin.

Acne assessment

Before taking measurements, subjects washed their faces using a standardised cleansing method and then acclimatised to the facility. The VISIA-CR system was used to capture facial images. Facial images were captured with the eyes closed and the face relaxed. Capture was performed in a resting position within a positioning system. The PRIMOS CR system was used to capture facial images in specific areas of interest, with subsequent analysis of acne lesions conducted using PRIMOS software. Standardised images of the participants' facial skin surface were obtained using the VISIA imaging system. Acne-related parameters (area, volume and height of facial acne) were analysed using VISIA software.

**EP 4 729 044 A1**

Clinical evaluation by a dermatologist

Clinical dermatologists performed a comprehensive visual and sensory assessment of the subjects' skin conditions. The test sites included the skin of the cheeks and forehead. At the time of enrolment in the study, written and witnessed informed consent was obtained from each subject explaining the objectives, risks and benefits of the study [15].

Statistical analysis

An experimental value was obtained from the selected region of interest (ROI) for each timepoint. For statistical analysis, all data collected before (D0) and after each control point (cp) were subjected to a normality test using the Shapiro-Wilk method [16].

The following methods were applied to assess the statistical significance of the T0 vs Tcp pairs. All variables that tested positive for normality (parametric variables) were analysed using the paired t-test method. All variables that did not test positive for normality (non-parametric) were analysed using the paired Wilcoxon method. The data were expressed as mean $\pm$ SD or SEM. For normalisation, the technical values obtained before (D0) and after treatment were relativised to the baseline values before treatment (D0) and expressed as a percentage.

Results and discussion

With microwave activation, the four NADES-based extracts were obtained in five minutes, with simple processing, showing interesting rheological properties (low viscosity) and a distinctive sensory profile.

Table I summarises the main results obtained for all NADES samples. Extraction efficiency was evaluated as the recovery of total phenolic content (TPC) and the antioxidant properties of the extracts as radical scavenging activity (RSA%). All samples showed high and stable antioxidant activity, consistent with TPC values ranging from 62 to 88%, with better results using betaine as a hydrogen bond acceptor.

Table I - Antioxidant activity (AA%) and total phenolic content (TPC) of selected NADES samples using DPPH and Folin-Ciocalteu assays.

| NADES Sample* | AA% $\pm$ SD | TPC (mg GAE/g) |
|---|---|---|
| CG | 77.8 $\pm$ 0.4 | 1.38 $\pm$ 0.07 |
| BG | 88.1 $\pm$ 0.1 | 1.72 $\pm$ 0.02 |
| CGA | 62.2 $\pm$ 1.0 | 1.12 $\pm$ 0.03 |
| BGA | 80.9 $\pm$ 0.3 | 1.57 $\pm$ 0.02 |

*Matrix/solvent ratio 1:10 w/w

As reported in Table II (MIC Test), both CGA and BGA samples revealed excellent antimicrobial potential against the Gram-positive (*S. aureus* and *S. epidermidis*) and Gram-negative (*P. aeuriginosa* and *E. coli*) MDR clinical strains tested here. In contrast, BG showed similar potency only against Gram-positive strains, while CG was found to have the lowest antibacterial power among the four NADES extracts tested, being active on Gram-positive strains mainly at concentrations of 6% v/v.

Table II - MIC values of NADES samples on Gram-negative bacterial strains

| Bacterial species | Strain | MIC %* | | | |
| | | CG | BG | CGA | BGA |
|---|---|---|---|---|---|
| *S. aureus* | 17 MRSA | 6 | 0.75 | 0.38 | 0.75 |
| | 18 MRSA | 6 | 0.38 | 0.38 | 0.38 |
| *S. epidermidis* | 2 MRSE | 6 | 0.75 | 0.38 | 0.38 |
| | 22 MRSE | 3 | 0.75 | 0.38 | 0.38 |
| *P. aeruginosa* | 259 CR +KAZ-AVI R | - | - | 0.38 | 0.38 |

6

(continued)

| Bacterial species | Strain | MIC %* | | | |
|---|---|---|---|---|---|
| | | CG | BG | CGA | BGA |
| *E. coli* | 477 CR (KPC) | - | - | 0.38 | 0.38 |

*v/v MRSA: Methicillin-resistant S. aureus; MRSE: Methicillin-resistant S. epidermidis CR: Carbapenem-resistant; KAZ-AVI R: Ceftazidime-avibactam-resistant; (KPC): Carbapenemase-producing Klebsiella pneumoniae

The biological activity results of CGA and BGA are comparable, but BGA also has excellent performance and a pleasant aroma, while in the case of CGA, choline chloride gives it a slight amine or 'fishy' odour.

GC-MS of its volatile fraction shows the presence of 21 volatile compounds, of which octadecanale (10.86%), eugenol (6.29%), linalool (4.33%), terpinen-4-ol (1.09%) and 1,8-cineole (0.96%) are the most representative compounds.

Differences in the assessment of skin acne for the twenty volunteers in terms of means and standard deviations were successfully obtained after three days of treatment. The results showed that the Active Cream significantly reduced the acne area by $7.2 \pm 3.7\%$ after only 3 days of treatment compared to baseline values (Table III). The improvement in acne volume is evident but not yet significant after 3 days, but we expect it to become significant after 28 days of treatment.

Tables III. Statistical parameters of the percentile data of all subjects examined with regard to acne characteristics (volume, area, height), measured at 3 days and 28 days. The means and standard deviations, both derived from all images, are indicated. Statistical significance is represented by an * for a p-value < 0.05.

## CLINICAL EVALUATION AFTER 3 DAYS OF TREATMENT

| Volume | D0 vs D3 CREAM with ACTIVE INGREDIENT - A | D0 vs D3 PLACEBO CREAM - B | D3 CREAM with ACTIVE INGREDIENT - A vs PLACEBO CREAM - B |
|---|---|---|---|
| Average difference | -7.2 | 1.5 | 8.7 |
| Standard Error | 4.1 | 4.5 | |
| p-value | 0.0404 | 0.7285 | 0.3983 |
| Summary | * | ns | ns |
| Significance | Yes | No | No |

| Area | D0 vs D3 CREAM with ACTIVE INGREDIENT - A | D0 vs D3 PLACEBO CREAM - B | D3 CREAM with ACTIVE INGREDIENT - A vs PLACEBO CREAM - B |
|---|---|---|---|
| Average difference | -6.9 | 1.8 | 8.7 |
| Standard Error | 3.9 | 4.0 | |
| p-value | 0.0342 | 1.0 | 0.2 |
| Summary | * | ns | ns |
| Significance | Yes | No | No |

| Height | D0 vs D3 CREAM with ACTIVE INGREDIENT - A | D0 vs D3 PLACEBO CREAM - B | D3 CREAM with ACTIVE INGREDIENT - A vs PLACEBO CREAM - B |
|---|---|---|---|
| Average difference | -0.1 | -0.7 | 0.6 |
| Standard Error | 0.8 | 0.8 | |

(continued)

| Height | D0 vs D3 CREAM with ACTIVE INGREDIENT - A | D0 vs D3 PLACEBO CREAM - B | D3 CREAM with ACTIVE INGREDIENT - A vs PLACEBO CREAM - B |
|---|---|---|---|
| p-value | 0.9237 | 0.4183 | 0.6011 |
| Report | ns | ns | ns |
| Significance | No | No | No |

**CLINICAL EVALUATION AFTER 28 DAYS OF TREATMENT**

| Volume | D0 vs D28 CREAM with ACTIVE INGREDIENT - A | D0 vs D28 PLACEBO CREAM - B | D28 CREAM with ACTIVE INGREDIENT - A vs PLACEBO CREAM - B |
|---|---|---|---|
| Average difference | -21.6 | 1.3 | 22.9 |
| Standard Error | 6.4 | 7.5 | |
| p-value | 0.0023 | 0.5459 | 0.0261 |
| Summary | ** | ns | # |
| Significance | Yes | No | Yes |

| Area | D0 vs D28 CREAM with ACTIVE INGREDIENT - A | D0 vs D28 PLACEBO CREAM - B | D28 CREAM with ACTIVE INGREDIENT - A vs PLACEBO CREAM - B |
|---|---|---|---|
| Average difference | -22.5 | 7.4 | 29.9 |
| Standard Error | 6.0 | 8.6 | |
| p-value | 0.0005 | 0.3884 | 0.0068 |
| Summary | *** | ns | ## |
| Significance | Yes | No | Yes |

| Height | D0 vs D28 CREAM with ACTIVE INGREDIENT - A | D0 vs D28 PLACEBO CREAM - B | D28 CREAM with ACTIVE INGREDIENT - A vs PLACEBO CREAM - B |
|---|---|---|---|
| Average Difference | 0.5 | -1.1 | 1.6 |
| Standard Error | 0.8 | 0.9 | |
| p-value | 0.6005 | 0.2079 | 0.3834 |
| Summary | ns | ns | ns |
| Significance | No | No | No |

Conclusion

Among green procedures, natural deep eutectic solvents (NADES) can be considered a rapid and effective extraction approach to improve the recovery of active compounds from Laurus nobilis leaves in a sustainable manner. The use of these eutectic systems as an alternative to organic solvents can align with the market trend towards natural products (since NADES themselves are composed of safe, naturally occurring molecules) and meet the requirements for skin functionality and efficacy in cosmetic compositions.

The combination of the extractive properties of NADES with the biological activity of eutectic solvents and *Laurus nobilis* results in synergistic performance of NaDES-based extracts. The result is a ready-to-use, antioxidant, self-preserving natural ingredient that can be added directly to a cosmetic formulation, providing overall functionality and anti-acne efficacy to the entire complex, as well as an interesting and unique olfactory character.

## References

1. Sharma, A., Singh, J., & Kumar, S. (2012). Laurel leaves. In K. V. Peter (Ed.), Handbook of herbs and spices (pp. 73-85). Woodhead Publishing Ltd.

2. Dobroslavić, E.; Repajić, M.; Dragović-Uzelac, V.; Elez Garofulić, I. Isolation of Laurus nobilis Leaf Polyphenols: A Review on Current Techniques and Future Perspectives. Foods 2022, 11, 235.

3. Caputo L, Nazzaro F, Souza LF, Aliberti L, De Martino L, Fratianni F, Coppola R, De Feo V. Laurus nobilis: Essential oil composition and its biological activities. Molecules. 2017 Jun 3;22(6):930.

4. Fidan H, Stefanova G, Kostova I, Stankov S, Damyanova S, Stoyanova A, Zheljazkov VD. Chemical composition and antimicrobial activity of Laurus nobilis L. essential oils from Bulgaria. Molecules. 22 February 2019;24(4):804.

5. WO11155829; WO23183775.

6. Caviglia, D., Russo, E., Preda, S., Robustelli della Cuna, F.S. and Villa, C. (2024), In situ NADES microwave-mediated extraction of bioactive compounds from Beta vulgaris L. var. rubra waste. Int J Food Sci Technol, 59: 3271-3282.

7. Singleton, V.L.; Orthofer, R.; Lamuela-Raventós, R.M. Analysis of total phenols and other oxidation substrates and antioxidants by means of Folin-Ciocalteu reagent. In Methods in Enzymology ; Academic Press Ltd; Elsevier Science Ltd: London, UK, 1999; Volume 299, pp. 152-178.

8. Russo, E., Spallarossa, A., Comite, A. et al. (2022). Valorisation and potential antimicrobial use of olive mill wastewater (OMW) from Italian olive oil production. Antioxidants, 11, 903.

9. Brand-Williams, W., Cuvelier, M.E. & Berset, C. (1995). Use of a free radical method to evaluate antioxidant activity. LWT - Food Science and Technology, 28, 25-30.

10. Singh, R.P., Chidambara Murthy, K.N. & Jayaprakasha, G.K. (2002). Studies on the antioxidant activity of pomegranate (Punica granatum) peel and seed extracts using in vitro models. Journal of Agricultural and Food Chemistry, 50, 81-86.

11. EUCAST European Committee on Antimicrobial Susceptibility Testing. [(accessed on 11 July 2022)]. Available online: https://www.eucast.org/ast_of_bacteria/].

12. R.P. Adams, Identification of Essential Oil Components by Gas Chromatography/Mass Spectrometry, 4th ed. Allured Publ. Corp, Carol Stream, IL, 2007

13. Cook C H, Centner R L, Michaels S E. An acne grading method using photographic standards. Arch. Dermatol. May 1979: 115: 571-575.

14. Burke B M, Cunliffe W J. The assessment of acne vulgaris-the Leeds technique. British Journal of Dermatology 1984: 111: 83-92.

15. Lucky A W, Barber B L, Girman C J, Williams J, Ratterman J, Waldstreicher J. A multirater validation study to assess the reliability of acne lesion counting. Journal of the American Academy of Dermatology 1996: 35(4): 559-565.

16. Lucky A W, Barber B L, Girman C J, Williams J, Ratterman J, Waldstreicher J. A multirater validation study to assess the reliability of acne lesion counting. Journal of the American Academy of Dermatology 1996: 35(4): 559-565.

## Claims

**1.** A composition comprising a ternary eutectic mixture and an extract of the laurel plant (*Laurus nobilis L.*), wherein the ternary mixture comprises glycerine or glycerol, lactic acid and at least one of betaine and choline chloride, and the extract of the laurel plant has a phenol content expressed in mg of gallic acid per gram of laurel, comprised between 10 and 100 mg/L, preferably between 20 and 80 mg/L.

**2.** The composition according to claim 1, wherein the weight ratio of choline chloride or betaine, glycerol and lactic acid is comprised between 1:1:1 and 3:1:1, preferably is 2:1:1.

**3.** The composition according to claim 1 or 2, wherein the extract is a laurel leaf extract.

**4.** The composition according to any one of the preceding claims, wherein the laurel leaf extract further comprises at least one of: octadecanale, preferably in an amount of between 8% and 11% by weight; eugenol, preferably in an amount of between 5% and 8%; linalool, preferably in an amount of between 2% and 8%; terpinen-4-ol, preferably in an amount of 1 to 5%; 1,8-cineole, preferably in an amount of 0.5 to 5%.

5. Process for preparing a composition according to any one of claims 1 to 4, comprising:

   a) bringing parts of the laurel plant, preferably laurel leaves, into contact with a ternary mixture of molecules forming a NADES eutectic solvent, said ternary mixture comprising glycerine or glycerol, lactic acid and at least one of betaine and choline chloride;
   b) heating the mixture of point a) to a temperature between 50ºC and 100ºC, preferably by microwave;
   c) separating the solid residue comprising the laurel leaf parts from the supernatant comprising a laurel leaf extract and the eutectic ternary mixture.

6. A cosmetic or pharmaceutical formulation comprising the composition according to any one of claims 1 to 4 and cosmetically and/or pharmaceutically acceptable excipients.

7. The formulation according to claim 6, in the form of creams, emulsions, oils, ointments, spray solutions.

8. The composition according to any one of claims 1 to 4 or the formulation according to claim 6 or 7, for use in the treatment of acne.

8. The composition according to any one of claims 1 to 4 or the formulation according to claim 6 or 7, for use as an antibacterial agent.

9. The composition according to any of claims 1 to 4 or the formulation according to claim 6 or 7, for use as an antioxidant.

**EUROPEAN SEARCH REPORT**

Application Number

EP 25 20 7653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Caviglia Debora ET AL: "Nades extractives from Laurus nobilis leaves: synergic functionalities and skin efficacy", , 1 January 2024 (2024-01-01), pages 1-1, XP093273477, Retrieved from the Internet: URL:https://iris.unige.it/handle/11567/1221639# [retrieved on 2025-04-29] * abstract * ----- | 1-3,6-10 | INV. A61K8/34 A61K8/365 A61K8/44 A61K8/9789 A61P17/10 A61Q19/00 |
| Y | KR 101 931 296 B1 (GREEN SOLUTION INC [KR]) 21 December 2018 (2018-12-21) * claims * ----- | 1-10 | |
| Y | WO 2023/218391 A1 (ACTIVE ITALIA S R L [IT]) 16 November 2023 (2023-11-16) * claims * ----- | 1-10 | |
| Y | WO 2023/026502 A1 (TOKIWA PHYTOCHEMICAL CO LTD [JP]) 2 March 2023 (2023-03-02) * claims * ----- | 1-10 | TECHNICAL FIELDS SEARCHED (IPC) A61K A61P A61Q |

The present search report has been drawn up for all claims

| Place of search | Date of completion of the search | Examiner |
|---|---|---|
| Munich | 10 March 2026 | Cismaru, L |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 7653

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2026

| Patent document cited in search report | | Publication date | Patent family member(s) | | Publication date |
|---|---|---|---|---|---|
| KR 101931296 | B1 | 21-12-2018 | NONE | | |
| WO 2023218391 | A1 | 16-11-2023 | EP | 4522148 A1 | 19-03-2025 |
| | | | US | 2025302904 A1 | 02-10-2025 |
| | | | WO | 2023218391 A1 | 16-11-2023 |
| WO 2023026502 | A1 | 02-03-2023 | EP | 4162944 A1 | 12-04-2023 |
| | | | JP | 7349742 B2 | 25-09-2023 |
| | | | JP | 2023031621 A | 09-03-2023 |
| | | | JP | 2023086841 A | 22-06-2023 |
| | | | JP | 2026020356 A | 06-02-2026 |
| | | | US | 2023330002 A1 | 19-10-2023 |
| | | | WO | 2023026502 A1 | 02-03-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 11155829 A

- WO 23183775 A

**Non-patent literature cited in the description**

- **SHARMA, A.** ; **SINGH, J.** ; **KUMAR, S.** Handbook of herbs and spices. Woodhead Publishing Ltd., 2012, 73-85
- **CAPUTO L** ; **NAZZARO F** ; **SOUZA LF** ; **ALIBERTI L** ; **DE MARTINO L** ; **FRATIANNI F** ; **COPPOLA R** ; **DE FEO V.** Laurus nobilis: Essential oil composition and its biological activities.. *Molecules.*, 03 June 2017, vol. 22 (6), 930
- **FIDAN H** ; **STEFANOVA G** ; **KOSTOVA I** ; **STANKOV S** ; **DAMYANOVA S** ; **STOYANOVA A** ; **ZHELJAZ-KOV VD.** Chemical composition and antimicrobial activity of Laurus nobilis L. essential oils from Bulgaria.. *Molecules.*, 22 February 2019, vol. 24 (4), 804
- **CAVIGLIA, D.** ; **RUSSO, E.** ; **PREDA, S.** ; **ROBUS-TELLI DELLA CUNA, F.S.** ; **VILLA, C.** In situ NADES microwave-mediated extraction of bioactive compounds from Beta vulgaris L. var. rubra waste.. *Int J Food Sci Technol*, 2024, vol. 59, 3271-3282
- Analysis of total phenols and other oxidation substrates and antioxidants by means of Folin-Ciocalteu reagent.. **SINGLETON, V.L.** ; **ORTHOFER, R.** ; **LAMUELA-RAVENTÓS, R.M.** In Methods in Enzymology. Academic Press Ltd, 1999, vol. 299, 152-178
- **RUSSO, E.** ; **SPALLAROSSA, A.** ; **COMITE, A. et al.** Valorisation and potential antimicrobial use of olive mill wastewater (OMW) from Italian olive oil production.. *Antioxidants*, 2022, vol. 11, 903

- **BRAND-WILLIAMS, W.** ; **CUVELIER, M.E.** ; **BER-SET, C.** Use of a free radical method to evaluate antioxidant activity.. *LWT - Food Science and Technology*, 1995, vol. 28, 25-30
- **SINGH, R.P.** ; **CHIDAMBARA MURTHY, K.N.** ; **JAYAPRAKASHA, G.K.** Studies on the antioxidant activity of pomegranate (Punica granatum) peel and seed extracts using in vitro models.. *Journal of Agricultural and Food Chemistry*, 2002, vol. 50, 81-86
- *EUCAST European Committee on Antimicrobial Susceptibility Testing.*, 11 July 2022, https://www. eucast.org/ast_of_bacteria
- **R.P. ADAMS**. Identification of Essential Oil Components by Gas Chromatography/Mass Spectrometry. Allured Publ. Corp, 2007
- **COOK C H** ; **CENTNER R L** ; **MICHAELS S E.** An acne grading method using photographic standards.. *Arch. Dermatol.*, May 1979, vol. 115, 571-575
- **BURKE B M** ; **CUNLIFFE W J.** The assessment of acne vulgaris-the Leeds technique.. *British Journal of Dermatology*, 1984, vol. 111, 83-92
- **LUCKY A W** ; **BARBER B L** ; **GIRMAN C J** ; **WILLIAMS J** ; **RATTERMAN J** ; **WALDSTREICHER J.** A multirater validation study to assess the reliability of acne lesion counting.. *Journal of the American Academy of Dermatology*, 1996, vol. 35 (4), 559-565